Europäisches Patentamt

European Patent Office

Office européen des brevets

(11) Publication number: 0 198 032
B1

# EUROPEAN PATENT SPECIFICATION

(45) Date of publication of patent specification: 03.01.90

(51) Int. Cl.⁵: C 12 N 5/00

(21) Application number: 85905265.6

(22) Date of filing: 09.10.85

(86) International application number:
PCT/US85/01948

(87) International publication number:
WO 86/02378 24.04.86 Gazette 86/09

(54) HOLLOW FIBER CELL CULTURE DEVICE AND METHOD OF OPERATION.

(30) Priority: 09.10.84 US 658549

(43) Date of publication of application:
22.10.86 Bulletin 86/43

(45) Publication of the grant of the patent:
03.01.90 Bulletin 90/01

(84) Designated Contracting States:
AT BE CH DE FR GB IT LI LU NL SE

(56) References cited:
US-A-3 407 120
US-A-3 734 851
US-A-3 911 140
US-A-3 997 396
US-A-4 220 725
US-A-4 266 026
US-A-4 391 912
US-A-4 440 853

(73) Proprietor: ENDOTRONICS INC.
8500 Evergreen Boulevard
Coon Rapids Minnesota 55433 (US)

(72) Inventor: CRACAUER, Ray, F.
3350 Oakland Avenue South
Minneapolis, MN 55407 (US)
Inventor: WALKER, Robert, D.
7531 Greenfield Avenue No. 103
Mounts View Minnesota 55432 (US)
Inventor: GRUENBERG, Micheal, L.
10855 Avocet
Coon Rapids, MN 55433 (US)

(74) Representative: Vossius & Partner
Siebertstrasse 4 P.O. Box 86 07 67
D-8000 München 86 (DE)

Courier Press, Leamington Spa, England.

Description

The present invention relates to cell culturing devices, and in particular, it relates to cell culturing devices having a plurality of hollow fiber membranes that efficiently and selectively transfer oxygen, nutrients and other chemical stimuli and remove waste products to grow and maintain cells *in vitro* at high cell densities to provide high yields of product formation per unit reactor volume.

Cell culture devices for culturing cells *in vitro* having a shell with a plurality of hollow fiber membranes have been known for quite some time. Medium containing oxygen, nutrients and other chemical stimuli is transported through the lumen of the hollow fiber membranes and undergoes a pressure drop resulting in an outward radial convective flow at the entry port of the device and an inward flow at the exit port of the device. Cells are grown in the fluid space between the fibers and the shall wall.

Hollow fiber culture devices have been proven to be ideal for the maintenance of many types of cells of high densities *in vitro*. The mass transfer characteristics of hollow fiber culture devices provide an efficient means of delivering nutrients and removing waste products from a culture. The semi-porous hollow fiber membranes can be selected with various pore sizes. With proper pore size selection, the cellular product can be maintianed on the outside of the fibers, while waste products and contaminating proteins will pass through the membrane pores into the lumen of the hollow fibers where they can be subsequently removed from the culture.

To economically produce cell-derived products in a hollow fiber culture device, large numbers of cells must be maintained viable in optimal culture conditions for product formation over long periods of time. Prior art hollow fiber culture devices have many limitations that prevent their use in the economical production of cell-derived products in commercial quantities. These limitations include: 1) formation of gradients in the cell compartment; 2) inability to directly monitor and control cellular environment; 3) lack of fluid movement in cell compartment leads to microenvironment formation around cells; 4) fibers are not equidistant apart in culture device leading to anoxic or dead spaces; 5) efficient mass transfer becomes difficult at high cell densities; and 6) the pressure drop across the device increases as the device is scaled up, increasing the problems cited above, thus limiting scaleability. The following explains each of the prior art limitations in more detail.

1. Formation of gradients in the cell compartment

Formation of gradients of nutrients, pH, $O_2$, $CO_2$, lactic acid, ammonia and other components of the culture media in the cell compartment is a common problem in hollow fiber culture devices. The gradients form due to the manner in which nutrient media flows through the device. Nutrients are more available to the cells near the inlet port due to the outward flow from the hollow fibers. As media flows to the outlet, metabolic waste products, such as lactic acid and ammonia, accumulate in the cell compartment undesirably affecting cell viability.

2. Inability to monitor and control cell compartment

Because of the nature of prior art hollow fiber cartridges, it is not possible to incorporate monitoring devices into the cell compartment. Thus, maintenance of optimal culture conditions is very difficult. The only control over the cell compartment environment is by diffusion of products from the lumen of the hollow fibers to the cell compartment.

3. Formation of microenvironments

Very little fluid motion occurs in the cell compartment of prior art hollow fiber culture devices. This leads to microenvironments forming around quickly metabolizing cells, adversely affecting other cells in the device by altering pH.

4. Formation of anoxic pockets

Hollow fibers are packed into cartridges. Usually the fibers are not equidistant apart and thus, some cells are farther away from the nutrient source than others. This leads to pockets where cells will die due to lack of oxygen or failure of nutrients to reach the cells by diffusion.

5. Mass transfer limitations

As the cells grow to high cell densities, nutrients must diffuse through greater layers of cells. Mass transfer by diffusion is limited, limiting the number of cells that can be maintained in the culture device.

6. Pressure drop

Hollow fiber culture devices perfuse cells in the cell compartment and remove waste products due to the forces set up by the pressure drop that occurs across the cartridge. This pressure drop becomes greater as the lengths of the fibers are increased. This pressure drop also creates the gradient problems described above. Thus, the length of the fibers is limited by the pressure drop which enhances the gradient problem.

Some examples of prior art cell culturing devices are described in the US patents 3734851, 3821087, 3883393, 3911140, 3997396, 4087327, 4184922, 4200689, 4201845, 4206015, 4220725, 4242460, 4266026, 4391912, 4396510, 4440853, and 4442206.

The purpose of the present invention is to overcome the above limitations, making it possible to utilize a hollow fiber culture device for the economical production of cell-derived products.

This object is achieved by the cell culture device and the method for culturing cells according to the claims.

The present invention includes an improved culturing device and method of culturing cells *in*

*vitro*. The device includes a hollow fiber cartridge having a shell with a plurality of capillaries extending therethrough with at least some of the capillaries having selectively permeable membrane walls. A first supply of medium is delivered to the capillaries at a first selected pressure and flow rate. A cell culturing space is defined between the shell and the capillaries. The culturing device further includes a chamber having a second supply of medium under a second selected pressure and being fluidly connected to the cell culturing space of the hollow fiber cartridge. The second supply of medium in the chamber is pressurized to a level greater than the medium flowing through the lumen of the capillaries. A valving mechanism is disposed to selectively and alternatively restrict the flow of the second supply of medium through first and second conduits, respectively, that connect the chamber with the cell culturing space such that circulation of fluid within the cell culturing space is affected.

The invention is described in detail in connection with the drawings in which:

Figure 1 is a diagrammatic view of an improved cell culturing device of the present invention.

Figure 2 is a diagrammatic view of an alternative embodiment of the cell culturing device of the present invention.

Figure 3 is a diagrammatical view of still another alternative embodiment of the cell culturing device of the present invention.

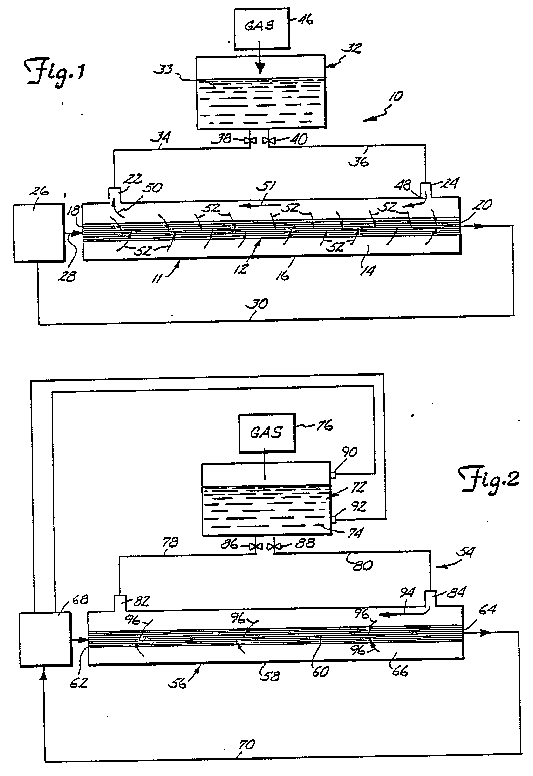

The apparatus of the present invention is diagrammatically illustrated in Figure 1. The improved cell culturing device includes a cell culturing unit such as a hollow fiber cartridge 11 in which cells are maintained and grown. The hollow fiber cartridge 11 includes a plurality of capillaries, generally indicated at 12 extending through a shell 16. Each capillary includes a lumen (not shown) through which medium containing oxygen, nutrients and other chemical components is transported. The oxygen, nutrients and other chemical components contained in the medium diffuse through membrane walls of the capillaries into an extracapillary space 14 containing cells which are to be maintained. The extracapillary space 14 is defined as the space between the shell 16 and the capillaries 12. Typically, the capillaries are disposed in a bundle which extends from an input end 18 to an output end 20 of the cartridge. A commercially-available cartridge suitabe for use in the device of the present invention is made by Amicon Corporation of Denvers, Massachusetts.

The cartridge 11 further includes first and second spaced-apart ports 22 and 24, respectively. Preferably, the first and second ports 22 and 24 are spaced apart so that the port 22 is located proximate the front end of the cartridge and the port 24 is located proximate the back end of the cartridge. Both ports are in fluid communication with the extracapillary space 14.

Types of cells that are suitable for *in vitro* culturing in the device of the present invention include bacteria, yeast and fungi that are naturally occurring or are modified by conjugation or genetic engineering techniques, such as transformation, DNA insertions, transduction, fusion and the like. In addition, primary and transformed mammalian cells (including mammalian cells altered by genetic engineering techniques and virus formation), tumor cells, hybridomas and other fused cells are well suited for culturing in the device of the present invention.

A delivery system 26 delivers a primary medium supply thorugh suitable conduit 28 to the lumen of the capillaries 12. The medium exits the cartridge into suitable tubing 30 that forms a recirculation line returning the medium back to the delivery system 26.

The delivery system 26 delivers medium at a selective rate and pressure. Suitable delivery systems are well known in the art.

An expansion chamber 32 is fluidly connected to the extracapillary space 14 through conduit 34 connected to the first port 22 and conduit 36 connected to the second port 24. First and second valves 38 and 40 are operably disposed to restrict flow between the extracapillary space 14 and the expansion chamber 32 within the conduits 34 and 36, respectively. In one working example, the conduits 34 and 36 include flexible tubing and the valves 38 and 40 are pinch-type valves which pinch the tubing to stop flow therethrough.

The expansion chamber 32 contains a second supply of medium 33. The medium supply 33 is selectively pressurized at a substantially constant level by a pressurizing system 46. In one working embodiment of the present invention, the pressurizing system 46 is a gas system that delivers an oxygen containing gas under pressure to the chamber 32.

The pressure in the expansion chamber 32 is kept at a higher pressure than the pressure within the lumens of the capillaries 12. The valves 38 and 40 are alternatively closed and opened to create circulation within the extracapillary space 14. For example, when valve 40 is opened and valve 38 is closed, flow of medium occurs through tubing 36 and into extracapillary space 14 through port 24, as indicated by arrow 48. Then valve 38 is opened and valve 40 is closed and flow occurs in the direction of arrow 50 through port 22 and through the tubing 34 into the expansion chamber 32. The positions of the valves are alternated such that circulation of fluid within the extracapillary space 14 is effected in the clockwise direction of arrow 51. Arrows 52 indicate the diffusion of waste products into the lumens of the capillaries 12. The circulation is in a pulsatile form, with the valves 38 and 40 being opened and closed momentarily.

Producing the circulation within the extracapillary space 14 eliminates the formation of gradients of nutrients, pH, $O_2$, $CO_2$, lactic acid, ammonia and other components in the extracapillary space that were a problem in the prior art. Microenvironments formed by quickly metabolizing cells are greatly minimized since circulation is effected in the extracapillary space.

In addition, circulation in the extracapillary space minimizes the gradient problems caused by the pressure drop that had occurred across prior art cartridges.

An alternative embodiment 54 of the device of the present invention is diagrammatically illustrated in Figure 2. The embodiment 54 similarly includes a hollow fiber cartridge 56 having a shell 58 and a plurality of semi-permeable capillaries 60 extending from the forward end 62 to a rearward end 64. An extracapillary space 66 is defined between the capillaries 60 and the shell 58. A delivery system 68 delivers a supply of medium at a selected pressure and flow rate to the lumens of the capillaries 60 similar to the delivery system 26 discussed previously. The medium is circulated from the outlet end 64 of the hollow fiber cartridge through tubing 70 back to the delivery system 68.

A chamber 72 contains a second supply of medium 74 that is pressurized at a substantially constant level by a gas pressurizing system 76. The chamber 72 is fluidly connected by tubing 78 and 80 to the extracapillary space 66 through ports 82 and 84, respectively. Valves 86 and 88 are operably connected to the conduits 78 and 80, respectively, so that flow may be selectively restricted in either the tubing 78 or 80.

The chamber 72 includes an upper level sensor 90 and a lower level sensor 92 that deliver a signal to the delivery system 68. When the upper sensor 90 senses a high level of medium within the chamber, the pressure under which the delivery system 68 is delivering medium to the capillaries 60 is lowered to a value less than the pressurization of the chamber 72 and sufficient to cause ultrafiltrative conditions. When a low level is sensed by the sensor 92, the delivery system increases the pressure that the medium is delivered under ultrafiltrative conditions through the capillaries to increase the amount of medium within the chamber 72.

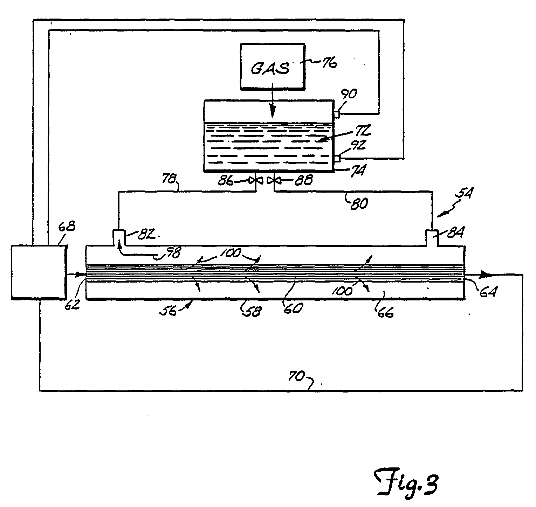

In operation, with valve 88 in the open position and valve 86 in the closed position, and the delivery system 68 delivering medium at a pressure less than the pressurization of the chamber 72, flow in the extracapillary space 66 is effected generally in the direction of arrow 94. Diffusion of waste products into the lumens of the capillaries 60 occurs as indicated by arrows 96. When the level within the chamber 72 falls past sensor 92, sensor 92 sends a signal to the delivery system 68 upon which the pressure of the medium being delivered to the capillaries 60 is raised above the level of pressurization within the chamber 72. The valve 86 is then opened and the valve 88 closed such that medium flows from the extracapillary space, through the port 82, into tubing 78 and back into the chamber 72, as initially indicated by arrow 98. Positive ultrafiltration perfusion of oxygen, $CO_2$, nutrients and other chemical components occurs through the walls of the capillaries 60, as indicated by arrow 100 in Figure 3.

The above sequence is alternated such that circulation is effected in the extracapillary space. In addition, diffusion of waste products and other undesirable components back into the lumen of the capillaries and diffusion of oxygen, $CO_2$, nutrients and other chemical components into the extracapillary space is enhanced.

In one example, the device of the present invention was used successfully to grow and maintain hybridoma cells that grew to cell densities of approximately $1 \times 10^8$ to $1 \times 10^9$ cells per milliliter and produced large quantities of a monoclonal antibody over a 90-day period. Typically, growing and maintaining hybridoma cells in a hollow fiber cartridge requires that the capillaries have membrane walls that have a selective molecular weight cut-off of less than approximately 50,000 Daltons. In the example, capillaries having a selective molecular weight cut-off of less than approximately 15,000 Daltons were used. The smaller molecular weight cut-off was used to significantly eliminate diffusion of the antibody being produced by the cells into the lumen of the capillaries. However, the decrease in the molecular weight cut-off increases resistance to oxygen transfer and nutrient transfer through the walls of the capillaries to the cells. In addition, there is increased resistance to waste product transfer (such as lactic acid produced by the cells) through the capillary walls into the lumen. A high waste product concentration is deleterious to the growth and maintenance of the cells.

It has been observed by the applicants in using the prior art hollow fiber cartridge arrangements, that an oxygen and nutrient concentration gradient formed across the length of the capillary bundle in the extracapillary space with a nutrient and oxygen build-up near the front end of the cartridge and a waste build-up near the back end of the cartridge. It is also believed that the build-up of nutrients and oxygen near the front end of the extracapillary space further added to the resistance of diffusion of oxygen and nutrients since the build-up decreases the concentration gradient across the capillary walls.

The expansion chamber had a medium volume approximately equal to the volume of the extracapillary space and was initially perssurized, for example, to a positive 100 millimeters of mercury (13.3 kPa). The valve 86 was opened while the valve 88 was closed. The delivery system 68 was operated to effect a pressure in the lumen of the capillaries of approximately 200 millimeters of mercury (26.6 kPa). The effect was that fluid flowed from the extracapillary space through tubing 78 into the expansion chamber and diffusion of $O_2$, $CO_2$, and nutrients into the extracapillary space.

When a high level of medium was sensed in the chamber, the pressure within the lumen of the capillaries was lowered below the pressure in the expansion chamber. The valve 88, previously closed, was opened while the valve 86, that was previously opened, was closed. The effect was that there was flow of medium from the expansion chamber 72 into the extracapillary space through tubing 80 and negative ultrafiltration of waste products.

The procedure was continued, alternating the relative pressure in the capillaries and alternating the position of the valves 86 and 88 to effect circulation of the medium through the cell culturing space 14 and positive ultrafiltration perfusion of desirable components into the extracapillary space and waste products out of the extracapillary space. It is believed that the effect of providing circulation in the cell culturing space enhances the environment in which the cells are grown. Nutrient and oxygen perfusion through the capillary membranes is increased when the pressure in the capillary lumen is greater than in the expansion chamber, and waste product diffusion through the capillary walls is enhanced when the pressure in the expansion chamber is greater than the pressure in the capillary lumen because the transfer is occurring under ultrafiltrative conditions. In addition, oxygen and nutrients are evenly distributed throughout the cell culturing space greatly increasing access to a much greater portion of the cells of both oxygen and nutrients.

It has also been observed that no significant difference in cell densities occurs whether a clockwise or counterclockwise circulation is effected between the extracapillary space 14 and the expansion chamber 32. A counterclockwise circulation is effected by closing valve 38 and opening valve 40 while the pressure in the expansion chamber 32 is less than the pressure in the lumen of the capillaries. When the pressure in the lumen of the capillaries is decreased while the pressure in the expansion chamber is increased to a level greater than in the lumen of the capillaries, valve 38 is open and valve 40 is closed.

The ultrafiltrative conditions created by the differential in presusre, both negative and positive, between the capillaries and the expansion chamber aids in the maintenance of high cell densities. Transport of oxygen and nutrients through the layers of cells is greatly enhanced. Removal of components that affect pH to the detriment of the cells is greatly increased under ultrafiltrative conditions.

## Claims

1. A cell culture device for in vitro cell growth, the device comprising a shell 16, 58 having first and second ends (18, 20; 62, 64), first and second ports (22, 24; 82, 84), and a plurality of capillaries (12; 60) extending between the first and second ends within the shell with at least some of said capillaries having selectively permeable walls, and a cell culturing space (14; 66) being defined between the shell and the capillaries with the first and second ports in fluid communication therewith, a first supply of medium being delivered into the capillaries at a first selected pressure and a second supply of medium for delivery at a second selected pressure higher than the first selected pressure, the device further comprising: a chamber (32; 72) fluidly connected to the first and second ports and containing the second supply of medium (33; 74), valving means (38, 40; 86, 88) for selectively restricting flow of the second supply of medium through the first and second ports into the culturing space, wherein the valving means is operated such that flow of the second supply of medium is alternated through the first and second ports to effect circulation within cell culturing space and enhance diffusion of waste products out of the culturing space and diffusion of medium components into the culturing space; pressurizing means (46; 76) for keeping the chamber at an approximately constant pressure; and wherein the delivery means is selectively controlled along with the valving means to raise and lower the pressure in the capillaries with respect to the pressure in the chamber effecting circulation within the cell culturing space.

2. The device according to claim 1 wherein upper and lower level sensors (90, 92) are disposed at a selected upper and lower level within the chamber 72 to sense a lower level and an upper level of medium; and wherein the delivery means (68) is selectively controlled along with the valving means to raise the pressure in the capillaries with respect to the pressure in the chamber when a low level is sensed and to lower the pressure in the capillaries with respect to the pressure in the chamber when a high level is sensed.

3. A cell culture device for in vitro cell growth, the device comprising: a hollow fiber cartridge (11; 56) having a shell (16; 58) with a plurality of capillaries (12; 60) extending between a fluid input end (18; 62) and a fluid output end (20; 64) and defining an extracapillary space (14; 66) between the shell, and the capillaries with at least some of said capillaries having selectively permeable walls and the shell having first and second spaced-apart ports (22, 24; 82, 84) communicating with the extracapillary space; delivery means (26; 68) for transporting to the capillaries and selectively raising and lowering pressure of a first supply of medium in the capillaries; a chamber (32; 72) fluidly connected to the first and second ports containing a second supply of medium (33; 74), valving means (38, 40; 86, 88) for selectively controlling flow of the second supply of medium from the chamber through the first and second ports; means (46; 76) for pressurizing the chamber; and wherein the delivery means alternatively raises and lowers the pressure in the capillaries above and below the pressure in the chamber in cooperation with the valving means so that circulation of medium within the extracapillary space is effected.

4. The device according to claim 3 wherein upper and lower level sensors (90, 92) are disposed to sense an upper and lower level of the medium within the chamber, respectively and

wherein the delivery means alternatively raises the pressure in the capillaries above the pressure in the chamber when a low level of medium is sensed in the chamber and lowers the pressure in the capillaries below the pressure in the chamber when a high level of medium is sensed in the chamber, said raising and lowering of pressure in the capillaries being done in cooperation with the valving means.

5. The device of claims 1 to 4 wherein the chamber (32; 72) is fluidly connected to the first and second ports (22, 24; 82; 84) of the shell by first and second conduits (34, 36; 78, 80) and wherein the valving means (38, 40; 86, 88) includes first and second valves controlling flow through the first and second conduits, respectively.

6. The device of claims 1 to 4 wherein the permeable walls of the capillaries have a selective molecular weight cut-off of less than approximatley 50,000 Daltons.

7. The device of claim 6 wherein the permeable walls of the capillaries (12; 60) have a selective molecular weight cut-off of less than approximately 15,000 Daltons.

8. The device of claims 1 to 7 wherein the pressurizing means (46; 76) includes a mechanism for delivering gas under pressure into the chamber.

9. A method for culturing cells in a hollow fiber cartridge having a shell and a plurality of capillaries extending through the shell, and defining a cell culturing space between the capillaries and shell with the cell culturing space being fluidly connected to an expansion chamber, the capillaries being provided with a first supply of medium by a delivery system and the expansion chamber being provided with a second supply of medium and including means for pressurizing the chamber, the method comprising:

alternatively increasing and decreasing the pressure within the capillaries above and below the pressure within the chamber; and

controlling the flow of the second supply of medium between the chamber and the extracapillary space by alternatively closing and opening first and second valves so that circulation is effected in the cell culturing space.

10. The method of claim 9 wherein the pressure is increased and decreased sufficiently to cause ultrafiltration of components from the medium through the capillary walls into the cell culturing space and ultrafiltration of components deleterious to cells in the cell culturing space into the lumen of the capillaries, respectively.

11. The method of claim 10 and further including establishing an upper level sensing point and a lower level sensing point and sensing when the medium has reached the upper level and the lower level; and increasing the pressure within the capillaries when the low level has been sensed and decreasing the pressure in the capillaries when the high level has been sensed.

**Patentansprüche**

1. Zellzüchtungsvorrichtung für das *in vitro*-Wachstum von Zellen, welche aufweist; ein Gehäuse (16; 58) mit einem ersten und einen zweiten Ende (18, 20; 62, 64), einer ersten und einer zweiten Öffnung (22, 24; 82, 84) und mehreren Kapillaren (12; 60), die sich zwischen dem ersten und zweiten Ende in dem Gehäuse erstrekken, wobei mindestens einige der Kapillaren selektiv permeable Wände aufweisen und ein Raum für die Zellzüchtung (14; 66) zwischen dem Gehäuse und den Kapillaren festgelegt wird, wobei die erste und die zweite Öffnung mit dem Raum in Fluidverbindung stehen, und ein erster Nährstoffvorrat einem ersten ausgewählten Druck in die Kapillaren zugeführt wird und ein zweiter Nährstoffvorrat bei einem zweiten ausgewählten Druck zugeführt wird, der höher als der erste ausgewählte Druck ist, und wobei die Vorrichtung ferner aufweist:

eine Kammer (32; 72), die mit der ersten und der zweiten Öffnung in Fluidverbindung steht und die den zweiten Nährstoffvorrat (33; 74) enthält;

eine Ventileinrichtung (38, 40; 86, 88) zum selektiven Steuern des Flusses des zweiten Nährstoffvorrats durch die erste und zweite Öffnung in den Züchtungsraum, wobei die Ventileinrichtung so arbeitet, daß der Fluß des zweiten Nährstoffvorrats abwechselnd durch die erste und zweite Öffnung verläuft, um eine Zirkulation innerhalb des Zellzüchtungsraumes zu bewirken und die Diffusion von Abfallprodukten aus dem Züchtungsraum sowie die Diffusion von Nährstoffkomponenten in den Züchtungsraum zu beschleunigen;

eine Druckeinrichtung (46; 76), um in der Kammer einen annähernd konstanten Druck zu halten;

une wobei die Zuführeinrichtung (68) zusammen mit der Ventileinrichtung selektiv so gesteuert werden, daß der Druck in den Kapillaren bezüglich des Druckes in der Kammer erhöht oder gesenkt wird, um die Zirkulation innerhalb des Zellzüchtungsraums zu bewirken.

2. Vorrichtung nach Anspruch 1, wobei Sensoren für ein oberes und ein unteres Niveau (90, 92) an einem ausgewählten oberen und unteren Niveau in der Kammer (72) angeordnet sind, um ein unteres und oberes Nährstoffniveau festzustellen, und wobei die Zuführeinrichtung (68) zusammen mit der Ventileinrichtung selektiv gesteuert werden, um den Druck in den Kapillaren bezüglich des Druckes in der Kammer zu erhöhen, wenn ein niedriges Niveau festgestellt wird, und den Druck in den Kapillaren bezüglich des Drukkes in der Kammer zu senken, wenn ein hohes Niveau festgestellt wird.

3. Zellzüchtungsvorrichtung für das *in vitro*-Wachstum von Zellen, welche aufweist:

eine Hohlfaserpatrone (11; 56) mit einem Gehäuse (16; 58) und mehreren Kapillaren (12; 60), die sich zwischen einem Fluideingangsende (18; 62) und einem Fluidausgangsende (20, 64) erstrecken und einen extrakapillaren Raum (14;

66) zwischen dem Gehäuse und den Kapillaren festlegen, wobei mindestens einige der Kapillaren mit selektiv permeablen Wänden versehen sind, und das Gehäuse eine erste und zweite voneinander beabstandete Öffnung (22, 24; 82, 84) aufweist, die mit dem extrakapillaren Raum in Verbindung stehen;

eine Zuführeinrichtung (26; 68), um einen ersten Nährstoffvorrat zu den Kapillaren zu transportieren und seinen Druck in den Kapillaren selektiv zu erhöhen oder zu senken;

eine Kammer (32; 72), die mit der ersten und zweiten Öffnung in Fluidverbindung steht und einen zweiten Nährstoffvorrat (33; 74) enthält;

eine Ventileinrichtung (38, 40; 86, 88) zum selektiven Steuern des Flusses des zweiten Nährstoffvorrats aus der Kammer durch die erste und zweite Öffnung;

eine Druckeinrichtung (46; 76) für die Kammer; und

wobei die Zuführeinrichtung in Zusammenwirken mit der Ventileinrichtung den Druck in den Kapillaren abwechselnd über und unter den Druck in der Kammer erhöht bzw. sentk, so daß eine Zirkulation des Nahrstoffs innerhalb des extrakapillaren Raum bewirkt wird.

4. Vorrichtung nach Anspruch 3, wobei Sensoren für ein oberes und unteres Niveau (90; 92) vorgesehen sind, um ein oberes bzw. unteres Niveau des Nährstoffs in der Kammer festzustellen, und wobei die Zuführeinrichtung in Zusammenwirken mit der Ventileinrichtung abwechselnd den Druck in den Kapillaren über den Druck in der Kammer erhöht, wenn ein niedriges Niveau des Nährstoffs in der Kammer festgestellt wird, und den Druck in den Kapillaren unter den Druck in der Kammer senkt, wenn ein hohes Nahrstoffniveau in der Kammer festgestellt wird.

5. Vorrichtung nach Anspruch 1 bis 4, wobei die Kammer (32; 72) mit der ersten und zweiten Öffnung (22, 24; 82, 84) des Gehäuses durch einen ersten und einen zweiten Kanal (34, 36; 78, 80) in Fluidverbindung steht, und wobei die Ventileinrichtung (38, 40; 86, 88) ein erstes und ein zweites Ventil aufweist, die den Fluß durch den ersten bzw. zweiten Kanal steuern.

6. Vorrichtung nach Anspruch 1 bis 4, wobei die permeablen Wände der Kapillaren (12; 60) eine selektive Molekulargewichtstrennung von weniger als etwa 50 000 Dalton aufweisen.

7. Vorrichtung nach Anspruch 6, wobei die permeablen Wände der Kapillaren (12; 60) eine selektive Molekulargewichtstrennung von weniger als etwa 15 000 Dalton aufweisen.

8. Vorrichtung nach Anspruch 1 bis 7, wobei die Druckeinrichtung (46; 76) einen Mechanismus zum Zuführen von unter Druck stehendem Gas in die Kammer aufweist.

9. Verfahren zur Zellzüchtung in einer Hohlfaserpatrone mit einem Gehäuse und mehreren Kapillaren, die sich durch das Gehäuse erstrecken, wobei ein Zellzüchtungsraum zwischen den Kapillaren und dem Gehäuse festgelegt ist, und wobei der Zellzüchtungsraum mit einer Expansionskammer in Fluidverbindung steht, die Kapillaren durch ein Zuführsystem mit einem ersten Nährstoffvorrat beschickt werden und die Expansionskammer mit einem zweiten Nährstoffvorrat beschickt wird und eine Druckeinrichtung vorgesehen ist, wobei das Verfahren aufweist:

abwechselndes Erhöhen und Senken des Drucks in den Kapillaren über oder unter den Druck in der Kammer; und

Steuern des Flusses des zweiten Nährstoffvorrats zwischen der Kammer und dem extrakapillaren Raum durch abwechselndes Schließen und Öffnen erster und zweiter Ventile, so daß eine Zirkulation im Zellzüchtungsraum bewirkt wird.

10. Verfahren nach Anspruch 9, wobei der Druck hinreichend erhöht und gesenkt wird, um sowohl ein Ultrafiltrieren der Komponenten aus dem Nährstoff durch die Wände der Kapillaren in den Zellzüchtungsraum als auch ein Ultrafiltrieren von für die Zellen im Zellzüchtungsraum schädlichen Komponenten in den Hohlraum der Kapillaren zu bewirken.

11. Verfahren nach Anspruch 10, welches ferner umfaßt, daß ein Abtastpunkt für ein oberes Niveau und ein Abtastpunkt für ein unteres Niveau festgelegt wird, und festgestellt wird, wenn der Nährstoff das obere Niveau und das untere Niveau erreicht hat, und daß der Druck in den Kapillaren erhöht wird, wenn das niedrige Niveau festgestellt wurde, und der Druck in den Kapillaren gesenkt wird, wenn das hohe Niveau festgestellt wurde.

**Revendications**

1. Dispositif pour la culture de cellules pour la croissance de cellules *in vitro*, le dispositif comportant une enceinte (16; 58) ayant une première et une seconde extrémités (18, 20; 62, 64), une première et une seconde ouvertures (22, 24; 82, 84) et une pluralité de capillaires (12; 60) s'étendant entre la première et la seconde extrémité dans l'enceinte, avec au moins certains capillaires ayant des parois sélectivement perméables, et un espace de culture de cellules (14; 66) étant défini entre l'enceinte et les capillaires, et étant en communication de fluide avec la première et la seconde ouverture, un premier approvisionnement d'agent étant transféré dans les capillaires à une première pression sélectionnée, et un second approvisionnement d'agent pour être transféré à une seconde pression sélectionnée supérieure à la première pression sélectionnée, le dispositif comportant en outre:

une chambre (32; 72) en connexion de fluide avec la première et la seconde ouvertures et contenant le second approvisionnement d'agent (33; 74);

des moyens de robinet (38, 40; 86, 88) pour limiter sélectivement le débit du second approvisionnement par la première et la seconde ouverture dans l'espace de culture, dans lequel les moyens de robinet sous actionnés de façon telle que le débit du second approvisionnement d'agent soit alterné dans la première et dans la seconde ouverture pour réaliser une circulation dans l'espace de culture de cellules et accroître la diffusion des déchets produits hors de l'espace de culture et la diffusion des constituants de l'agent dans l'espace de culture;

des moyens de mise en pression (46; 76) pour maintenir la chambre à une pression sensiblement constante; et

dans lequel le moyen de transfert (68) est sélectivement commandé en même temps que les moyens de robinet pour élever et abaisser la pression dans les capillaires par rapport à la pression dans la chambre effectuant la circulation dans l'espace de culture de cellules.

2. Dispositif selon la revendication 1, dans lequel des capteurs de niveaux supérieur et inférieur (90, 92) sont disposés à des niveaux sélectionnés supérieur et inférieur dans la chambre (72) pour détecter un niveau supérieur et un niveau inferieur de l'agent, et dans lequel le moyen de transfert (68) est commandé sélectivement en même temps que les moyens de robinet pour élever la pression dans les capillaires par rapport à la pression dans la chambre quand un niveau inférieur est détecté et pour abaisser la pression dans les capillaires par rapport à la pression dans la chambre quand un niveau supérieur est détecté.

3. Dispositif de culture de cellules pour la croissance in vitro des cellules, le dispositif comprenant:

une cartouche (11; 56) de fibres creuses ayant une enceinte (16; 58) avec une pluralité de capillaires (12; 60) s'étendant entre une extrémité d'entrée de fluide (18; 62) et une extrémité de sortie de fluide (20; 64) définissant un espace extracapillaire (14; 66) entre l'enceinte et les capillaires avec au moins certains desdits capillaires ayant des parois sélectivement perméables et l'enceinte ayant une première et une seconde ouvertures espacées l'une de l'autre (22, 24; 82, 84) en communication avec l'espace extracapillaire;

un moyen de transfert (26; 68) pour transférer aux capillaires un premier approvisionnement d'agent et en élever et abaisser sélectivement la pression dans les capillaires;

une chambre (32; 72) en connexion de fluide avec la première et la seconde ouverture, contenant un second approvisionnement d'agent (33; 74);

des moyens de robinet (38, 40; 86, 88) pour commander sélectivement le débit du second approvisionnement d'agent de la chambre par la première et la seconde ouverture;

des moyens (46; 76) pour mettre en pression la chambre; et

dans lequel le moyen de transfert élève et abaisse alternativement la pression dans les capillaires au-dessus et au-dessous de la pression dans la chambre en coopération avec les moyens de robinet de façon que la circulation de l'agent dans l'espace extracapillaire soit assurée.

4. Dispositif selon la revendication 3, dans lequel

des capteurs de niveaux supérieur et inférieur (90, 92) sont disposés pour détecter respectivement un niveau supérieur et un niveau inférieur de l'agent dans la chambre; et

dans lequel le moyen de transfert élève alternativement la pression dans les capillaires audessus de la pression dans la chambre quand un niveau inférieur d'agent est détecté dans la chambre et abaisse la pression dans les capillaires au dessous de la pression dans la chambre quand un niveau supérieur d'agent est détecté dans la chambre, lesdits élèvement et abaissement de pression dans les capillaires étant faits en coopération avec les moyens de robinet.

5. Dispositif selon une des revendications 1 à 4, dans lequel la chambre (32; 72) est en connexion de fluide avec les première et seconde ouvertures (22, 24; 82, 84) de l'enceinte par des premier et second conduits (34, 36; 78, 80) et dans lequel les moyens de robinet (38, 40; 86, 88) comprennent un premier et un second robinets qui commandent le débit respectivement dans le premier et le second conduits.

6. Dispositif selon une des revendications 1 à 4, dans lequel les parois perméables des capillaires (12; 60) ont une coupure sélective de poids moléculaire inférieure à approximativement 50,000 Daltons.

7. Dispositif selon la revendication 6, dans lequel les parois perméables des capillaires (12; 60) ont une coupure sélective de poids moléculaire inférieure approximativement à 15,000 Daltons.

8. Dispositif selon une des revendications 1 à 7, dans lequel les moyens de mise en pression (46, 76) comportent un mécanisme pour transférer du gaz sous pression dans la chambre.

9. Procédé pour cultiver des cellules dans une cartouche de fibres creuses ayant une enceinte et une pluralité de capillaires s'étendant à travers la cartouche, et définissant un espace de culture de cellules entre les capillaires et l'enceinte, dans lequel l'espace de culture de cellules est en connexion de fluide avec une chambre d'expansion, les capillaires étant alimentés d'un premier approvisionnement d'agent par un système de transfert et la chambre d'expansion étant alimentée d'un second approvisionnement d'agent et comportant des moyens pour mettre en pression la chambre, le procédé comportant:

accroître et décroître alternativement la pression dans les capillaires au-dessus et endessous de la pression dans la chambre; et

commander le débit du second approvisionnement entre la chambre et l'espace extracapillaire en ferment et ouvrant alternativement les premier et second robinets de façon que la circulation soit assurée dans l'espace de culture de cellules.

10. Procédé selon la revendication 9, dans lequel la pression est accrue et décrue suffisamment pour causer respectivement l'ultrafiltration de composants à partir de l'agent à travers les parois des capillaires vers l'espace de culture de cellules et l'ultrafiltration de composants nuisibles aux cellules dans l'espace de culture de cellules vers le lumen des capillaires.

11. Procédé selon la revendication 10 comportant en outre l'etablissement d'un point de détection de niveau supérieur et d'un point de détection de niveau inférieur et détecter quand l'agent à atteint le niveau supérieur et le niveau inférieur; et accroître la pression dans les capillaires quand le niveau inférieur a été atteint, et décroître la pression dans les capillaires quand le niveau supérieur a été atteint.

9

Fig.1

Fig.2

1

Fig.3